# EUROPEAN PATENT SPECIFICATION

(11) **EP 1 392 863 B1**
(45) Date of publication and mention of the grant of the patent: **23.04.2008**
(21) Application number: 02727638.5
(22) Date of filing: 06.06.2002
(51) Int. Cl.: C12Q 1/68

(54) **METHOD FOR DETERMINING THE PRESENCE OF EXTENSION PRODUCTS**
BESTIMMUNGSVERFAHREN FÜR DAS VORHANDENSEIN VON VERLÄNGERUNGSPRODUKTEN
PROCEDE SERVANT A DETERMINER LA PRESENCE DE PRODUITS D'EXTENSION

(30) Priority: 06.06.2001 FI 20011195; 12.06.2001 US 297264 P
(43) Date of publication of application: 03.03.2004
(73) Proprietor: BIOHIT OYJ, 00880 Helsinki (FI)
(72) Inventor: ORPANA, Arto, FIN-00670 HELSINKI (FI); SUOVANIEMI, Osmo, FIN-00570 HELSINKI (FI)
(74) Representative: Karvinen, Leena Maria
(86) International application number: PCT/FI2002/000489
(87) International publication number: WO 2002/099127

(56) References cited:
- WO-A1-00/11220
- WO-A1-01/81631
- WO-A1-92/14137
- WO-A1-99/28500
- WO-A2-01/04357
- US-A- 5 876 936
- SYVANEN ANN-CHRISTINE ET AL.: 'From gels to chips: 'minisequencing' primer extension for analysis of point mutations and single nucleotide polymorphisms' HUMAN MUTATION vol. 13, 1999, pages 1 - 10, XP000953256
- CHEN XIANGNING ET AL.: 'Fluorescence energy transfer detection as a homogeneous DNA diagnostic method' PROC. NATL. ACAD. SCI. USA vol. 94, 1997, pages 10756 - 10761, XP002927445

## Description

### Field of the invention

The present invention relates to a method for determining extension products in reactions involving nucleic acid chain extension, e.g., in cyclic minisequencing, and to the use of this method when identifying specific point mutations and genetic variations.

### Background of the invention

Point mutations, changes of a single nucleotide in a gene, are a major cause of human inherited diseases. For diagnostic purposes there have been multiple technologies available for detection of known point mutations. These methods can be classified as being based either on differential hybridisation kinetics between fully and partially complementary DNA strands or on enzyme-assisted point mutation detection (Landegren, et al., 1998).

The classical ASO (allele specific oligonucleotide hybridisation) performed with radioactively labelled oligonucleotide probes (Wallace, et al., 1979), which has over the years developed into a kinetic homogenous assay systems (Whitcombe, et al., 1998; Wittwer, et al., 1997a; Wittwer, et al., 1997b) demonstrates the rapid developments of this field. Study of hundreds or thousands of mutations became possible when it was shown that high-density DNA arrays can be synthesized directly on a glass surface (Fodor, et al., 1991; Lipshutz, et al., 1999). This rapidly growing DNA micro array technology is also based on the differential hybridisation between fully and partially complementary DNA strands.

The enzyme-assisted methods are usually based either on DNA polymerase or DNA ligase (Barany, 1991; Landegren, et al., 1988; Wallace, et al., 1979) enzyme activities. The original point mutation assay performed to a single sample (Syvanen, et al., 1990) (Ihalainen, et al., 1994) has developed to a multiplexed assay performed in array format (Kurg, et al., 2000; Pastinen, et al., 1997; Pastinen, et al., 1996; Pastinen, et al., 2000; Shumaker, et al., 1996) and can be used for detection of point mutations or single nucleotide polymorphisms (SNPs). In all of these systems the detectable reaction product is formed only if in the assay environment the test probe is fully complementary to the target sequence (Syvanen, 1999). In the case of DNA polymerase based assays the reaction product is formed only if a nucleoside triphosphate complementary to the sequence in the template DNA is available in the reaction mixture.

There are multiple well-characterized tools for detection of known point mutations today, but the price of a single test is relatively high. This has become a major concern during the latest developments in genetic research, as it is becoming evident that the differences between individuals are caused by small differences such as SNPs, randomly located in every 1 to 2 kb of our genome (Sachidanandam, et al., 2001). Already at least 1.4x10⁶ SNPs are available in public databases (Sachidanandam, et al., 2001), and the number will grow over time, and SNPs are becoming a powerful tool of genetic and biomedical research. These differences are very usefull also in identification and fine mapping of disease causing or predisposing genes. Especially multifactorial diseases are currently studied using SNP genotyping. One of the main problems with the use of a SNP for genotyping is that alone a SNP is not informative enough, and for efficient identification of e.g. the alleles associated with a disease a set of several SNPs located in a narrow segment of the chromosome is needed (SNP haplotype), which results in that more SNP markers than traditional markers must be studied (Dawson, 1999; Isaksson, et al., 2000; Martin, et al., 2000) and very sophisticated algorithms must be developed (Morris, et al., 2000) before one can get reliable results.

The most commonly used methods for finding of these single nucleotide differences has been direct DNA sequencing of the polymorphic area of a gene, most often after amplification of genomic DNA or mRNA isolated from the target tissue by the polymerase chain reaction (PCR). However, due to the availability of huge amounts of raw sequencing data collected during the large scale human sequencing projects, a great deal of SNPs have been identified *in silico* by comparing available sequences obtained from multiple individuals.

One of the major difficulties in obtaining sufficient amounts of SNP data is the complexity of current methods used for their detection as well as their high price. One of the major costs of a SNP genotyping method is the modification of oligonucleotide primers used in these assays. Labelling or other modifications of oligonucleotide primers, usually coupling to an anchoring or fluorescent detection unit, together with mandatory purification steps after these coupling reactions, increase the cost of a single oligonucleotide. If hundreds of modified oligonucleotides are required for a single project this becomes a major cost over time. The most attractive possibility, the use of DNA array technology, has very high up-front cost and when the array is printed no changes in the assay format and SNP repertoire can be performed without re-printing the slides.

For this reason an inexpensive, robust and simple method of the invention for the determination of the presence of extension products, ie. extended oligonucleotide primers, in reactions involving nucleic acid chain extension was developed. The invention is also related to an efficient way to determine results of cyclic minisequencing used, e.g., for the detection of SNPs. The major advantage of the novel method described here is that, when a SNP is analysed, only three inexpensive standard unmodified primers are needed, two for PCR amplification to amplify the area of interest in the genomic DNA of a patient, and one for cyclic minisequencing (cMS) to examine the specific position (the SNP) in the template nucleic acid derived from the preceding PCR amplification. If detection of the SNP is performed in both strands, only a single additional unmodified primer is needed. The method of the invention is flexible allowing studying SNPs in any order and SNPs can be added to or removed from the overall scheme at will. This is important when fine mapping an area of interest. Only minor optimisations or modifications are needed when testing various SNPs. The detection is based on fluorescence detection and can be done with a standard plate fluorometer and it is compatible for complete automation of the liquid handling steps.

The method of the invention is also related to a method utilizing fluorescence resonance energy transfer (FRET) which refers to distance-dependent interaction between the electronically excited states of two dye molecules in which excitation energy is transferred from a donor molecule to an acceptor molecule without emission of a photon. Primary conditions for FRET are: (i) donor (source) and acceptor (target) molecules must be in close proximity (typically 10-100 Å), (ii) the absorption spectrum of the acceptor must overlap the fluorescence emission spectrum of the donor. Probes labeled with such energy transfer coupled dyes are described, e.g., in US patent No. 6,028,190.

Fluorescence has proven to be a versatile tool for a myriad of applications. It is a powerful technique for studying molecular interactions in analytical chemistry, biochemistry, cell biology, physiology, nephrology, cardiology, photochemistry, and environmental science. It boasts phenomenal sensitivity for the analytical chemist or the life scientist working at nanomolar concentrations. But fluorescence offers much more than mere signal-gathering capability. New developments in instrumentation, software, probes, and applications have resulted in a burst of popularity for a technique that was first observed over 150 years ago.

As the theoretical underpinnings of fluorescence became more understood, a more powerful set of applications emerged that yield detailed information about complex molecules and their reaction pathways. The binding of biochemical species can be easily studied *in situ.* Distances within macromolecules may be measured. The dynamics of the folding of proteins can be studied. Concentrations of ions can be measured inside living cells. Membrane structure and function may be studied with fluorescence probes. Drug interactions with cell receptors can be investigated. Minute traces of fluorescent materials can be detected and identified in mixtures. Oil samples can be finger-printed and identified by their fluorescence. The electronic structure and dynamics of an excited state of a molecule may be elucidated. These are only a few examples of the applications of modem fluorescence techniques.

Fluorescence is a phenomenon in which absorption of light of a given wavelength by a fluorescent molecule is followed by the emission of light at longer wavelengths. The distribution of wavelength-dependent intensity that causes fluorescence is known as the fluorescence excitation spectrum, and the distribution of wavelength-dependent intensity of emitted energy is known as the fluorescence emission spectrum. Fluorescence detection has three major advantages over other light-based investigation methods: high sensitivity, high speed, and safety. The point of safety refers to the fact that samples are not affected or destroyed in the process, and no hazardous byproducts are generated. Sensitivity is an important issue because the fluorescence signal is proportional to the concentration of the substance being investigated. Relatively small changes in ion concentration in living cells may have significant physiological effects. Whereas absorbance measurements can reliably determine concentrations only as low as several tenths of a micromolar, fluorescence techniques can accurately measure concentrations one million times smaller, pico- and even femtomolar. Quantities less than an attomole (<10⁻¹⁸ mole) may be detected.

By using fluorescence, one can monitor very rapid changes in concentration. Changes of picosecond order in fluorescence intensity can be detected, if necessary. Because it is a non-invasive technique, fluorescence does not interfere with a sample. The excitation light levels required to generate a fluorescence signal are low, reducing the effects of photo-bleaching. Thus, living tissue can be investigated with no adverse effects on its natural physiological behavior.

The technique of vertical photometry is commonly used with microplate instruments. Not only because it enables a matrix type of plate to be measured but provides also some interesting benefits. The most important feature of vertical photometry is that the absorbance is not a function of the concentration of the sample, but only depends on the amount of the absorbing substance in the cuvette. Therefore the effect of evaporation or layering is practically eliminated. The same technique is nowadays commonly in use with microplate fluorometers with same benefits.

If the movement of the fluorescent molecules in the sample is restricted to the extent that the orientation of the electric dipole oscillator responsible for the emission is the same or bears a fixed relationship to the dipole oscillator responsible for the absorption of the excited photon, the emission light is polarized even if the excitation light is unpolarized. If the sensitivity of the detection system is dependent on the polarization state of the emission, errors in fluorescence intensity measurement may occur.

On the other hand, time dependence of anisotropy may provide important information for the study of excited states. Details of electronic transitions and the microenvironment of a fluorophore can be gained from the steady state fluorescence anisotropy. Anisotropy (or polarization) measurements yield information on molecular movements that take place during the fluorescence lifetime. It is not surprising, then, that measuring the time-dependent decay of fluorescence anisotropy is even more informative regarding rotational and diffusive motions of macromolecules.

The design of vertically measuring fluorometers is described in Suovaniemi (1994), Tiusanen (1992) and Harjunmaa (1986).

### Summary of the invention

The present invention relates to a method of determining the presence of extension products, ie. an extended oligonucleotide primer, in a reaction mixture, wherein a deoxynucleoside (5'-)triphosphate (dNTP) to be template-dependently incorporated to the 3' end of an oligonucleotide primer comprises a label, comprising the steps of:
a) extending said oligonucleotide primer with said dNTP under conditions suitable for primer extension;
b) contacting said extended oligonucleotide primer derived from step (a) with a single-stranded DNA specific fluorescent dye for binding said extended oligonucleotide primer;
c) determining the presence of said extended oligonucleotide primer by measuring fluorescence emission intensity after excitation.

The present invention also provides a method of determining the presence of extension products, ie. an extended oligonucleotide primer, in a reaction mixture, wherein a template-dependently incorporated nucleotide in the 3' end of said extended oligonucleotide primer comprises a fluorescent detection moiety, comprising the steps of:
a) treating said reaction mixture to obtain single stranded extension products, or if the extension products are already single stranded, directly employing step (b);
b) contacting the single-stranded extension products in said reaction mixture with a single-stranded DNA specific fluorescent dye for binding close to the 3' end of the extended oligonucleotide primer so as to form an energy transfer relationship between said fluorescent detection moiety and said fluorescent reagent to provide for fluorescence resonance energy transfer (FRET) between said fluorescent detection moiety and said single-stranded DNA specific fluorescent dye;
c) determining the presence of said extended oligonucleotide primer by measuring fluorescence emission intensity after excitation.

The present invention also provides a method of determining the presence of extension products, ie. an extended oligonucleotide primer, in a reaction mixture, wherein a template-dependently incorporated nucleotide in the 3' end of said extended oligonucleotide primer comprises a separation moiety, comprising the steps of:
a) treating said reaction mixture to obtain single stranded extension products, or if the extension products are already single stranded, directly employing step (b);
b) separating said extended oligonucleotide primer from said reaction mixture;
c) contacting said extended oligonucleotide primer separated in step (b) with a single-stranded DNA specific fluorescent dye for binding to said extended oligonucleotide primer;
d) determining the presence of said extended oligonucleotide primer by measuring the fluorescence intensity after excitation.

The present invention also relates to cyclic minisequencing and to the detection of point mutations and genotyping single nucleotide polymorphisms (SNPs).

### A brief description of the drawings

Fig.1 shows the ratio between the emission at 635 nm and at 535 nm of the ssDNA specific fluorescence reagent (OliGreen™) bound to cMS primers, when different cMS primer concentrations were used.

Fig. 2 shows a schematic drawing describing the steps of Example 1. rxn, reaction; w/, with; w/o, without; F-dNTP, dNTP with fluorescence label for mutant (mut) or wild type (WT) allele.

Fig. 3 shows the results when patient samples were screened by the method of Example 1 for the test SNP, factor V Leiden mutation, Arg506Gln. Control samples for normal genotype, homo- and heterozygous mutant genotypes as well as blank samples (H₂O) are shown.

Fig. 4 shows the results when patient samples were screened by the method of Example 2 for the test SNP, factor V Leiden mutation, Arg506Gln. Control samples for normal genotype, homo- and heterozygous mutant genotypes as well as blank samples (H₂O) are shown.

Fig. 5 shows fluorescence measurement geometries when the sample vessel is e.g. a microplate cuvette. Thick black arrow presents the direction of the excitation signal. Thin grey arrows present the direction of the emission signals. Four different configurations are shown (a, b, c and d)

### Detailed description of the invention

The following describes a method for determining extension products in reactions involving nucleic acid chain extension and use thereof in SNP detection procedure.

The first step of a SNP screening procedure of the invention is a standard PCR amplification or RT-PCR amplification of a patient DNA or RNA sample, respectively, for the mutation locus using a specific primer pair. Then the PCR primers and dNTPs are degraded by a Exo I - SAP -treatment, as in the great majority of the detection systems today. Of course, there are alternative methods for the removal of primers and dNTPs from a completed polymerase reaction, e.g. different filtering methods. The next step is a template dependent extension of an unmodified cyclic minisequencing (cMS) primer using dNTP labelled with a fluorescence dye, such as Texas Red^{™}, in a cyclic primer extension reaction. Due to the cyclic labelling of the detection primer, the labelling efficiency is higher than in conventional solid phase minisequencing, leading to a high signal to noise ratio. In the cyclic minisequencing step the template nucleic acid derived from the preceding PCR step is subjected to parallel primer extension reactions having the same specific cMS primer, but each reaction comprising only one type of labelled dNTP. The types of dNTPs, e.g. dATP, dCTP, dGTP or dTTP, chosen for each reaction depend on the variation in the examined nucleotide position. The primer hybridizes to the template so that the 3' end of the primer binds to a nucleotide immediately adjacent to the examined nucleotide position. The solid phase minisequencing method is described in detail in US patent No. 6,013,431, and the advantages of cyclic primer extension reaction, when screening specific DNA sequences, are discussed in US patent No. 5,710,028.

As a dNTP is used, inexpensive standard thermostabile DNA polymerase enzymes can be used in the cMS step. The extended and thus labelled primer, for instance, by Texas Red^{™}, is then detected by fluorescence resonance energy transfer (FRET) based system after adding an oligonucleotide or single strand DNA (ssDNA) specific fluorescence dye (such as OliGreen™, Molecular Probes) to completed cMS reactions. Due to the short distance between the two dyes bound to the extended primer, the fluorescence energy coming from the oligonucleotide or single strand DNA (ssDNA) specific fluorescence dye, applied to and evenly distributed to the cMS primer, is transferred to the fluorescence label of that nucleotide, which was incorporated to the 3' end of the primer during the primer extension reaction. This fluorescence label acts as a fluorescent detection moiety in the method of the invention. If the used fluorescent detection moiety is Texas Red^{™} dye, the amount of fluorescence intensity in a reaction is directly related to the amount of incorporated Texas Red^{™} -labelled nucleotide. This is controlled by the DNA polymerase's stringent selection of acceptable nucleotide based on the template DNA sequence, and thus by the sequence in the original template DNA.

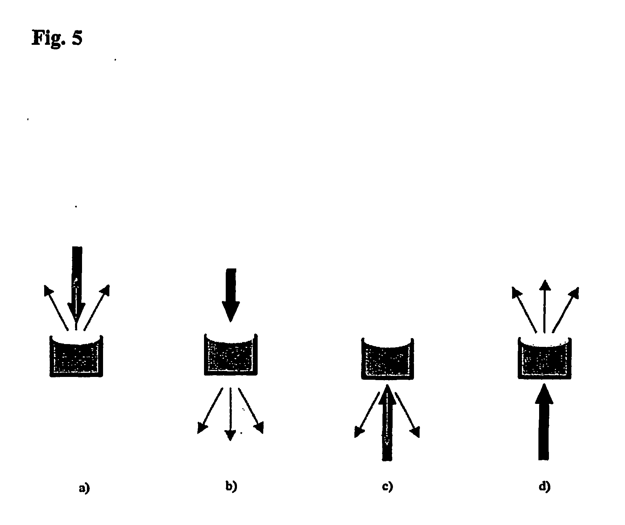

The described method of the invention showed excellent differentiation of the genotypes. However, there are always slight technical differences in the pipetting volumes, when preparing primer extension reactions, and this can cause technical variation. The disturbing effect of this variation can be bypassed using the following strategy when measuring the results of the present method. The sensitivity of the method increases when, instead of measuring only the emission signal of the fluorescent detection moiety, the emission signals from both the fluorescent detection moiety and the single strand DNA specific fluorescence dye are measured, and the ratio between them is then calculated for the determination of the results. The measurement with more than two wavelengths from the emission spectra of the two dyes is also useful when calculating the quantity of the energy transfer. The fluorescence intensity results can be obtained by excitating the sample in a sample vessel through the side of the uncovered liquid surface or through the bottom side of the sample vessel and measuring the emitted radiation from the side of the excitation. Alternatively, the fluorescence intensity results are obtained by measuring the emitted radiation from the side opposite to the side of the excitation. The sample vessel can be, e.g., a microplate cuvette or a solid support. The fluorescence measurement geometries are shown in Figure 5. In principle, the isotropically emitted fluorescent light can be measured at almost any angle to the direction of the excitation signal. Furthermore, the angle of the excitation signal to the sample vessel or sample surface can vary.

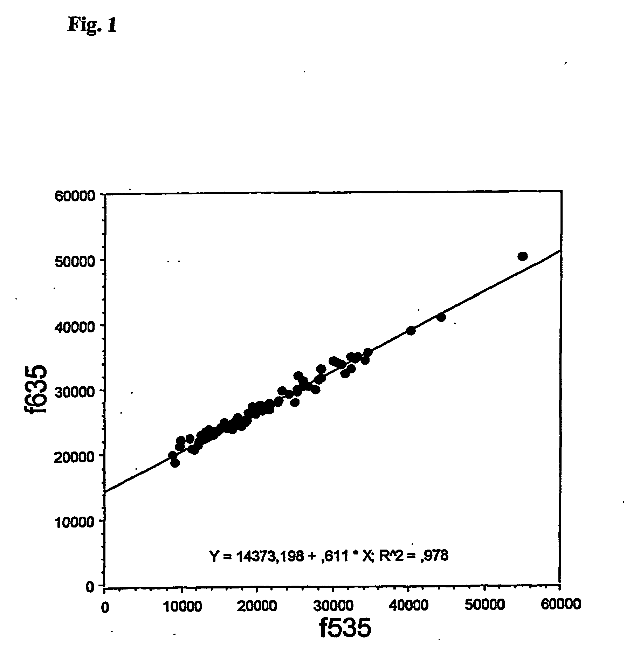

In the case of Example 1, OliGreen™ was the single strand DNA specific fluorescence dye and Texas Red^{™} dye was used as fluorescent detection moiety, and they were acting as donor fluorophore and acceptor fluorophore, respectively. OliGreen™ dye has absorption maximum at about 498 nm and emission maximum at about 518 nm, and Texas Red^{™} dye has absorption maximum at about 596 nm and emission maximum at about 620. So, in order to determine the ratio between the Texas Red^{™} emission and OliGreen™ emission intensities, the signals were measured with the wavelengths 635 nm and 535 nm, respectively. Any change in the ratio of 635/535 nm was due to FRET to Texas Red^{™} label from OliGreen™ dye, as, in the absence of extended products and Texas Red^{™} label, the ratio of OliGreen™ emission intensities at those wavelengths remained constant in the presence of various amounts of oligonucleotides in a reaction (Fig. 1).

An advantage of the method of the invention is that quality control is easier than in conventional methods, as one can synthesize a standard unmodified oligonucleotide mimicing the template strand and introduce the polymorphism of interest during the oligo synthesis, and then use this oligo as template instead of amplified patient DNA. Thus, one of the common problems of SNP genotyping, obtaining heterozygous control DNA for testing assay performance can be bypassed by providing a single oligonucleotide.

The principle of using FRET in point mutation detection and SNP genotyping was presented by Chen and Kwock already a few years ago and both DNA ligase (Chen, et al., 1998) and DNA polymerase (Chen and Kwok, 1997; Chen, et al., 1997) have been used in these homogenous assays. However, the key finding of the present work is that the nonspecific ssDNA dye can be used as one of the fluorophores in the FRET assay. This has two major advantages. Firstly as only inexpensive unmodified oligonucleotides are used in the assay, the cost of the analysis of a single SNP decreases dramatically. Secondly, unlike conventional systems, wherein a single fluorescein molecule is attached to the 5' end of the detection oligonucleotide, in this assay the fluorescent dye is advantageously distributed along the oligonucleotide back bone. Thus, multiple source fluorochromes are available and the critical factor in FRET assay, the optimal distance between the source and target fluorochromes, is achieved.

Fluorescence polarization (FP) can also be used for SNP detection (Chen, et al., 1999; Gibson, et al., 1997), and also this method requires no modified oligonucleotides and the assay format is very similar to the method of the invention. In this assay the FP of a dye-labelled ddNTP terminator changes due to the attachment to the detection primer. The major differences between FP- and FRET are that a specific fluorometer capable of FP measurement must be used, and much more care must be taken to optimize the PCR and Exo-SAP steps to completely remove the dNTPs from the PCR. The reason for this is that the amount of fluorescent-labelled dye must be low to be able to detect the change in the FP and even then the change in the FP signal is relatively low which makes the assay vulnerable to technical errors. In the method of the invention the signal-to-noise ratio is high allowing more reliable genotyping.

This invention is also related to a method, wherein the first step of a SNP screening procedure of the invention is a standard PCR amplification of the mutation locus using a specific primer pair and the primers and dNTPs are degraded in a Exo I-SAP -treatment, as presented above. This can also be done, e.g., by different filtering methods. The next step, however, is a template dependent extension of an unmodified cMS primer with biotinylated dNTP in a cyclic primer extension reaction, such as a minisequencing reaction. The extended and thus biotinylated primer is then separated, e.g., by trapping the primer to a solid support, such as the bottom of a 384-well microtitre plate which has a Neutravidin^{™} (Pierce) or streptavidin coating with a high affinity to biotin molecules. Thus, biotin can be used as a separation moiety in the present method. After washing away the unbound primers, the amount on bound primer is measured simply by adding a single strand DNA (ssDNA) specific fluorecence dye. The amount of fluorescence intensity in a reaction is directly related to the amount of incorporated biotinylated nucleotide which is controlled by the DNA polymerase's stringent selection of acceptable nucleotide based on the template DNA sequence, and thus by the sequence in the original template DNA.

### High sensitivity in detection of ssDNA labeled with fluorescent dyes

Typically, the Stoke's shift separating the optimal excitation and emission wave lengths is around 20 - 40 nm. For most equipment used for fluorescence detection the optical system has difficulties in completely avoiding leakage of excitation signal resulting in increased background and decreased sensitivity of the detection. We have observed that very high detection sensitivity of e.g. Cy5™ labeled oligos can be achieved by using OliGreen™ assisted FRET. In the presence of OliGreen™ excitation can be performed at 485 nm and detection at 667 nm. This nearly 200 nm difference makes it possible to use very large slits for both excitation and emission, resulting in very high sensitivity of detection.

A primer extension reaction for the detection of single nucleotide differences between DNA templates can be used for many applications. Disease causing mutations or disease predisposing polymorphisms is one field to utilise the present invention. Most PHenotype differences between individuals are caused by single nucleotide polymorphisms (SNPs) and already millions of SNPs have been identified. Moreover, exact strains, or species of animals, bacteria or virus, or even plants can be identified by studying single nucleotide differences. Detection of single base differences can also be used for evaluation of resistance to drugs or antibiotics. In microbiology as well as in pharmacogenomics, SNPs causing altered drug response or metabolism are now extensively studied.

The present invention is illustrated with the following examples, which are not intended to limit the scope of the invention.

### Example 1

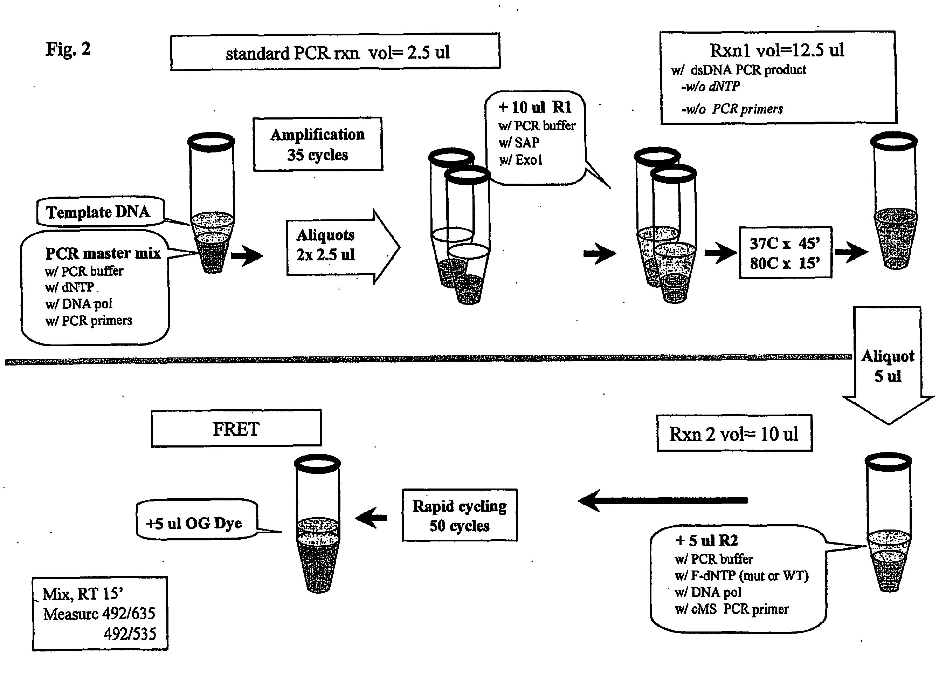

The SNP genotype was determined by cycle minisequencing (cMS) technique modified from the original solid phase minisequencing (US patent No. 6,013,431). A schematic drawing describing the steps of Example 1 is presented in Fig. 2. DNA to be used as a template was extracted from the frozen EDTA blood samples using conventional methods. The primers for the PCR amplification were flanking the polymorphic SNP site, in this case the Factor V Leiden mutation (Arg506Gln, corresponding to the nucleotide change from G to A in the coding DNA strand of the genome) conferring protein C resistance and increased trombosis risk in a patient. The sequences of the primers were 5'-TCT CTT GAA GGA AAT GCC CCA-3' and 5'-GGG CTA ATA GGA CTA CTT CTA-3'. Five µL of template DNA (5 ng/µL) was added to the 40 µL PCR Mastermix pre-aliquoted on 96 well PCR plates. The PCR amplification was performed in the reaction buffer of the DNA polymerase supplemented with 25 µM PCR primers, 200 µM dNTP, and 0.5 units of the thermostabile DNA polymerase. The amplification was performed with a 64 °C ⇒ 58°C Touch down PCR program in a MJ Research Tetrad cycler by cycling for 35 times: at 96°C for 1 min, progressively decreasing annealing temperature from 64°C to 58 °C for 1 min and at 72 °C for 2 min, after an initial 5 min incubation at 96 °C.

After PCR amplification, unreacted dNTPs and primers were removed by adding 0.2 unit of shrimp alkaline phosphatase (SAP) and 1 unit of Exonuclease I (EXO I) in 10 µL of the reaction buffer of the DNA polymerase to 2.5 µl of the PCR reaction. The degradation of primers and dNTPs was performed at 37°C for 45 min, after which the enzymes were inactivated by incubation at 80°C for 15 min.

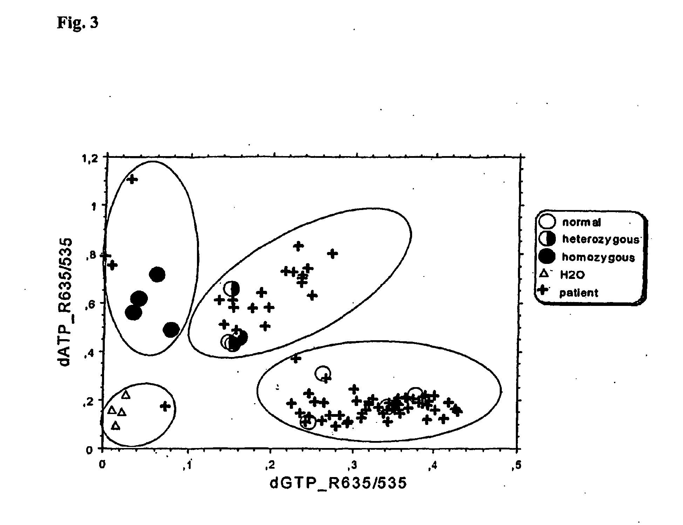

Two aliquots, 5 µl each from the enzyme treated PCR product were transferred on new PCR plates (Thermo-Fast 384, ABgene, Epsom, Surrey). After that 5 µl of the primer extension master mix containing 5 pmols of the cMS primer 5'-GAG CAG ATC CCT GGA CAG GC-3', 0.5 U thermostabile DNA polymerase and 10 pmoles of Texas Red™-labelled dGTP or dATP were added, one labelled dNTP per aliquot, for the detection of the WT and mutated alleles, respectively. The plate was covered with the plate sealer (Microseal 384, MJ Research), and the cyclic primer extension was performed by cycling at 96°C for 10 seconds and at 56°C for 10 seconds, for 50 cycles. After cycling, the amount of extended cMS primer was quantitated by fluorescence energy resonance transfer (FRET) system after adding 5 µl of single stranded DNA specific fluorescent dye OliGreen™ (Molecular Probes, Eugene OR) diluted 1:50 with TE buffer (10 mM TRIS- HCl, pH 7.0, 1mM EDTA). The plate was incubated at room temp for 10 min with constant shaking. The fluorescence intensity of the samples were measured by excitation at 485 nm and emission at 535 and 635 nm. The 635/535 nm ratio was used for genotyping. If the cMS primer was extended during the cycling step, fluorescence emission energy from the OliGreen™ was transferred to the TexasRed™ dye due to the close distance, resulting in elevated 635/535 nm emission ratio. As shown in Fig. 3, the samples studied for the test SNP, factor V Leiden mutation Arg506Gln, show excellent differentiation between genotypes.

### Example 2

DNA to be used as a template was extracted from the frozen EDTA blood samples using conventional methods. The primers for the PCR amplification were flanking the polymorphic SNP site, in this case the Factor V Leiden mutation (Arg506Gln, corresponding to the nucleotide change from G to A in the coding DNA strand of the genome) conferring protein C resistance and increased trombosis risk in a patient. The sequences of the primers were 5'-TCT CTT GAA GGA AAT GCC CCA-3' and 5'-GGG CTA ATA GGA CTA CTT CTA-3'. Five µL of template DNA (5 ng/µL) was added to the 40 µL PCR Mastermix pre-aliquoted on 96 well PCR plates. The PCR amplification was performed in reaction buffer of the DNA polymerase supplemented with 25 µM PCR primers, 200 µM dNTP, and 0.5 units of the thermostabile DNA polymerase. The amplification was performed with a 64 °C ⇒ 58°C Touch down PCR program in a MJ Research Tetrad cycler, cycling at 96 °C for 1 min, progressively decreasing annealing temperature from 64 °C to 58°C for 1 min and at 72°C for 2 min, after an initial 5 min incubation at 96 °C. After PCR amplification, unreacted dNTPs and primers were removed by adding 0.2 unit of shrimp alkaline phosphatase (SAP) and 1 unit of Exonuclease I (EXO I) in 10 µL of the reaction buffer of the DNA polymerase to 2.5 µl of the PCR reaction. The degradation of primers and dNTPs was performed at 37°C for 45 min, after which the enzymes were inactivated by incubation at 80°C for 15 min.

Two aliquots, 5 µl each from the enzyme treated PCR product were transferred on new PCR plates (Thermo-Fast 384, ABgene, Epsom, Surrey). After that 5 µl of the primer extension master mix containing 5 pmols of the cMS primer 5'-GAG CAG ATC CCT GGA CAG GC-3', 0.5 U thermostabile DNA polymerase and 10 pmoles of biotinylated dGTP or dATP were added, one labelled dNTP per aliquot, for the detection of the WT and mutated alleles, respectively. The plate was covered with the plate sealer (Microseal 384, MJ Research), and the cyclic primer extension was performed by cycling at 96°C for 10 seconds and at 56 °C for 10 seconds, for 50 cycles.

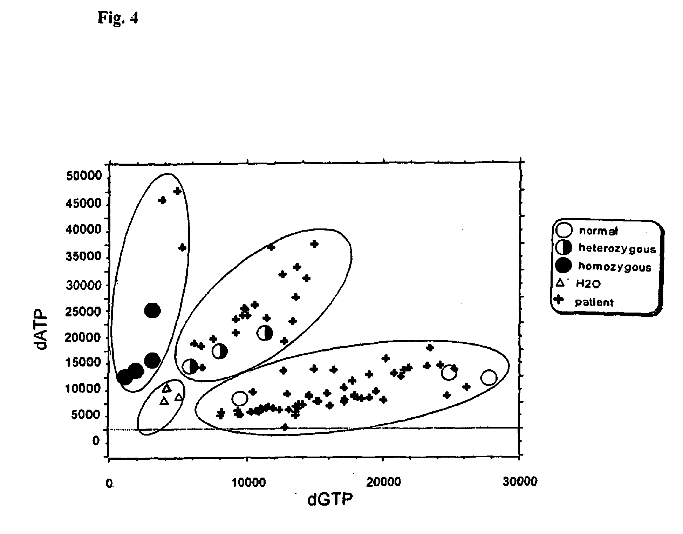

After cycling, the whole reaction mix was transfered into wells of black 384 streptavidin -coated micro plates (384 black NeutrAvidin™ with Superblocker^{™}, Pierce). The plates were incubated at 37 °C for one hour to allow binding of the biotinylated primer into the wells. The plates were washed in a plate washer, after which the 50 µl of single stranded DNA specific fluorescent dye OliGreen™ (Molecular Probes, Eugene OR) diluted 1:250 with TE buffer (10 mM TRIS- HCl, pH 7.0, 1mM EDTA). The plate was incubated at room temp for 10 min with constant shaking and the amount of cMS primer attached to the well was quantitated by measuring fluorescence at excitation at 485 nm and emission at 535 nm. As shown in Fig. 4, the samples studied for the test SNP, factor V Leiden mutation Arg506Gln, show excellent differentiation between genotypes.

### Example 3

We tested various fluorescent dyes for their use as an acceptor of FRET energy from ssDNA binding fluorescent reagent OliGreea™ (Molecular Probes, Eugene, OR). We added 5 µl of single stranded DNA specific fluorescent reagent OliGreen™ diluted 1:50 with TE buffer (10 mM TRIS- HCl, pH 7.0, 1mM EDTA) into a sample containing 5 pmols of a labeled primer having one of the following dyes: tetramethyl rhodamine (emission at around 552 nm), Texas Red^{™} (emission at around 620 nm, Molecular Probes, Eugene OR), Cy3™ (emission at around 570 nm, Amersham Life science, Inc.), or Cy5™ (emission at around 667 nm, Amersham Life Science, Inc.). All of these dyes emitted at their expected emission wavelength when attached to an oligonucleotide and exited at 485 nm in the presence of OliGreen™ bound to the primer. Thus, with ssDNA binding fluorescent reagent and FRET, one can detect the presence of multiple oligonucleotides carrying fluorophores with different emission peaks.

### References

Barany, F. (1991) Genetic disease detection and DNA amplification using cloned thermostable ligase, Proceedings of the National Academy of Sciences of the United States of America, 88, 189-93.
Chen, X. and P.Y. Kwok (1997) Template-directed dye-terminator incorporation (TDI) assay: a homogeneous DNA diagnostic method based on fluorescence resonance energy transfer, Nucleic Acids Research, 25, 347-53.
Chen, X., L. Levine and P.Y. Kwok (1999) Fluorescence polarization in homogeneous nucleic acid analysis, Genome Research, 9, 492-8.
Chen, X., K.J. Livak and P.Y. Kwok (1998) A homogeneous, ligase-mediated DNA diagnostic test, Genome Research, 8, 549-56.
Chen, X., B. Zehnbauer, A. Gnirke and P.Y. Kwok (1997) Fluorescence energy transfer detection as a homogeneous DNA diagnostic method, Proceedings of the National Academy of Sciences of the United States of America, 94, 10756-61.
Dawson, E. (1999) SNP maps: more markers needed? [news], Molecular Medicine Today, 5, 419-20.
Fodor, S.P., J.L. Read, M.C. Pirrung, L. Stryer, A.T. Lu and D. Solas (1991) Light-directed, spatially addressable parallel chemical synthesis, Science, 251, 767-73.
Gibson, N.J., H.L. Gillard, D. Whitcombe, R.M. Ferrie, C.R. Newton and S. Little (1997) A homogeneous method for genotyping with fluorescence polarization, Clinical Chemistry, 43, 1336-41.
Harjunmaa H. (1986) Theory, design and immunological applications of a vertically measuring fluorometer, doctoral thesis, University of Helsinki.
Ihalainen, J., H. Siitari, S. Laine, A.C. Syvanen and A. Palotie (1994) Towards automatic detection of point mutations: use of scintillating microplates in solid-phase minisequencing., Biotechniques, 16, 38-43.
Isaksson, A., U. Landegren, A.C. Syvanen, P. Bork, C. Stein, F. Ortigao and A.J. Brookes (2000) Discovery, scoring and utilization of human single nucleotide polymorphisms: a multidisciplinary problem, European Journal of Human Genetics, 8, 154-6.
Kurg, A., N. Tonisson, I. Georgiou, J. Shumaker, J. Tollett and A. Metspalu (2000) Arrayed primer extension: solid-phase four-color DNA resequencing and mutation detection technology, Genetic Testing, 4, 1-7.
Landegren, U., R. Kaiser, J. Sanders and L. Hood (1988) A ligase-mediated gene detection technique, Science, 241, 1077-80.
Landegren, U., Nilsson M. and Kwok, P-Y (1998) Reading bits of genetic information: Methods for single-nucleotide polymorphism analysis, Genome Research, 8, 769-776.
Lipshutz, R.J., S.P. Fodor, T.R. Gingeras and D.J. Lockhart (1999) High density synthetic oligonucleotide arrays, Nature Genetics, 21, 20-4.
Martin, E.R., E.H. Lai, J.R. Gilbert, A.R. Rogala, A.J. Afshari, J. Riley, K.L. Finch, J.F. Stevens, K.J. Livak, B.D. Slotterbeck, S.H. Slifer, L.L. Warren, P.M. Conneally, D.E. Schmechel, I. Purvis, M.A. Pericak-Vance, A.D. Roses and J.M. Vance (2000) SNPing away at complex diseases: analysis of single-nucleotide polymorphisms around APOE in Alzheimer disease, American Journal of Human Genetics, 67, 383-94.
Morris, A.P., J.C. Whittaker and D.J. Balding (2000) Bayesian fine-scale mapping of disease loci, by hidden Markov models, American Journal of Human Genetics, 67, 155-69.
Pastinen, T., A. Kurg, A. Metspalu, L. Peltonen and A.C. Syvanen (1997) Minisequencing: a specific tool for DNA analysis and diagnostics on oligonucleotide arrays, Genome Research, 7, 606-14.
Pastinen, T., J. Partanen and A.C. Syvanen (1996) Multiplex, fluorescent, solid-phase minisequencing for efficient screening of DNA sequence variation, Clinical Chemistry, 42, 1391-7.
Pastinen, T., M. Raitio, K. Lindroos, P. Tainola, L. Peltonen and A.C. Syvanen (2000) A system for specific, high-throughput genotyping by allele-specific primer extension on microarrays, Genome Research, 10, 1031-42.
Sachidanandam, R., D. Weissman, S.C. Schmidt, J.M. Kakol, L.D. Stein and M. G. (2001) A map of human genome sequence variation containing 1.42 million single nucleotide polymorphisms, Nature, 409, 928-33.
Shumaker, J.M., A. Metspalu and C.T. Caskey (1996) Mutation detection by solid phase primer extension, Human Mutation, 7, 346-54.
Suovaniemi O. (1994) Automated instrumentation for clinical and research laboratories, doctoral thesis, University of Helsinki.
Syvanen, A-C (1999) From gels to chips: "Minisequencing" primer extension for analysis of point mutations and single nucleotide polymorphisms, Human Mutation, 13, 1-10.
Syvanen, A-C, K. Aalto-Setala, L. Harju, K. Kontula and H. Soderlund (1990) A primer-guided nucleotide incorporation assay in the genotyping of apolipoprotein E, Genomics, 8, 684-92.
Tiusanen, T. (1992) Inner-filter correction with a fluorometer-based multifunctional instrument, doctoral thesis, University of Helsinki.
Wallace, RB., J. Shaffer, R.F. Murphy, J. Bonner, T. Hirose and K. Itakura (1979) Hybridization of synthetic oligodeoxyribonucleotides to phi chi 174 DNA: the effect of single base pair mismatch, Nucleic Acids Research, 6, 3543-57.
Whitcombe, D., J. Brownie, H.L. Gillard, D. McKechnie, J. Theaker, C.R. Newton and S. Little (1998) A homogeneous fluorescence assay for PCR amplicons: its application to real-time, single-tube genotyping, Clinical Chemistry, 44, 918-23.
Wittwer, C.T., M.G. Herrmann, A.A. Moss and R.P. Rasmussen (1997a) Continuous fluorescence monitoring of rapid cyclic DNA amplification, Biotechniques, 22, 130-1, 134-8.
Wittwer, C.T., K.M. Ririe, R.V. Andrew, D.A. David, R.A. Gundry and U.J. Balis (1997b) The LightCycler: a microvolume multisample fluorimeter with rapid temperature control, Biotechniques, 22, 176-81.

## Claims

1. A method of determining the presence of extension products, i.e. an extended oligonucleotide primer, in a reaction mixture, wherein a deoxynucleoside (5'-)triphosphate (dNTP) to be template-dependently incorporated to the 3' end of an oligonucleotide primer comprises a label, comprising the steps of:
a) extending said oligonucleotide primer with said dNTP under conditions suitable for primer extension;
b) contacting said extended oligonucleotide primer derived from step (a) with a single-stranded DNA specific fluorescent dye for binding to said extended oligonucleotide primer;
c) determining the presence of said extended oligonucleotide primer by measuring fluorescence emission intensity after excitation.

2. A method of determining the presence of extension products, i.e. an extended oligonucleotide primer, in a reaction mixture, wherein a template-dependently incorporated nucleotide in the 3' end of said extended oligonucleotide primer comprises a fluorescent detection moiety, comprising the steps of:
a) treating said reaction mixture to obtain single stranded extension products, or if the extension products are already single stranded, directly employing step (b);
b) contacting the single-stranded extension products in said reaction mixture with a single-stranded DNA specific fluorescent dye for binding close to the 3' end of the extended oligonucleotide primer so as to form an energy transfer relationship between said fluorescent detection moiety and said single-stranded DNA specific fluorescent dye to provide for fluorescence resonance energy transfer (FRET) between said fluorescent detection moiety and said single-stranded DNA specific fluorescent dye;
c) determining the presence of said extended oligonucleotide primer by measuring the fluorescence intensity after excitation.

3. The method as in claim 2, wherein said energy transfer relationship is based on energy transfer between a donor fluorophore and an acceptor fluorophore.

4. The method as in claim 2, wherein the presence of said extended oligonucleotide primer is determined in step (c) by measuring with more than one wavelength from the emission spectra of said fluorescent detection moiety and said single-stranded DNA specific fluorescent dye.

5. The method as in claim 2, wherein said single-stranded DNA specific fluorescent dye, acting as a donor fluorophore, has an absorption maximum at about 498 nm and an emission maximum at about 518 nm, and said fluorescent detection moiety, acting as an acceptor fluorophore, has an absorption maximum at about 596 nm and an emission maximum at about 620.

6. The method as in claim 2, wherein only one nucleotide is incorporated in the 3' end of said extended oligonucleotide primer.

7. The method as in claim 2, wherein said reaction mixture contains the necessary components for cyclic minisequencing.

8. The method as in claim 2, wherein said reaction mixture contains the necessary components for cyclic minisequencing and the template in said reaction mixture is a PCR or RT-PCR product derived from a patient sample.

9. The method as in claim 2, which is used to confirm point mutations, for instance SNPs, allelism, or the genotype of human or animal being.

10. The method as in claim 2, wherein said template-dependently incorporated nucleotide in the 3' end of the extended oligonucleotide primer is derived from dATP, dCTP, dGTP or dTTP present in said reaction mixture and labelled with a fluorescent dye.

11. The method as in claim 2, wherein the presence of said extended oligonucleotide primer is determined in step (c) by excitating the reaction mixture sample in a sample vessel through the side of the uncovered liquid surface or through the bottom side of said sample vessel and measuring the emitted radiation from the side of the excitation.

12. Themethod as in claim 2, wherein the presence of said extended oligonucleotide primer is determined in step (c) by excitating the reaction mixture sample in a sample vessel through the side of the uncovered liquid surface or through the bottom side of said sample vessel and measuring the emitted radiation from the side opposite to the side of the excitation.

13. A method of determining the presence of extension products, ie. an extended oligonucleotide primer, in a reaction mixture, wherein a template-dependently incorporated nucleotide in the 3' end of an extended oligonucleotide primer comprises a separation moiety, comprising the steps of:
a) treating said reaction mixture to obtain single stranded extension products, or if the extension products are already single stranded, directly employing step (b);
b) separating said extended oligonucleotide primer from said reaction mixture;
c) contacting said extended oligonucleotide primer separated in step (b) with a single-stranded DNA specific dye for binding to said extended oligonucleotide primer;
d) determining the presence of said extended oligonucleotide primer by measuring the fluorescence intensity after excitation.

14. The method as in claim 13, wherein said extended oligonucleotide primer is immobilized in step (b) to a solid support coated with a specific receptor for said separation moiety.

15. The method as in claim 13, wherein biotin is used as said separation moiety and said extended oligonucleotide primer is immobilized in step (b) to a solid support coated with streptavidin or avidin.

16. The method as in claim 13, wherein the presence of said extended oligonucleotide primer is determined in step (c) by measuring with more than one wavelength from the emission spectrum of said single-stranded DNA specific dye.

17. The method as in claim 13, wherein said single-stranded DNA specific fluorescent dye has an absorption maximum at about 498 nm and an emission maximum at about 518 nm.

18. The method as in claim 13, wherein only one nucleotide is incorporated in the 3' end of said extended oligonucleotide primer.

19. The method as in claim 13, wherein said reaction mixture contains necessary components for cyclic minisequencing.

20. The method as in claim 13, wherein said reaction mixture contains necessary components for cyclic minisequencing and the template in said reaction mixture is a PCR or RT-PCR product derived from a patient sample.

21. The method as in claim 13, which is used to confirm point mutations, for instance SNPs, allelism, or the genotype of human or animal being.

22. The method as in claim 13, wherein said template-dependently incorporated nucleotide in the 3' end of the extended oligonucleotide primer is derived from dATP, dCTP, dGTP or dTTP present in said reaction mixture and labelled with biotin.

23. The method as in claim 13, wherein the presence of said extended oligonucleotide primer is determined in step (d) by excitating the reaction mixture sample in a sample vessel through the side of the uncovered liquid surface or through the bottom side of said sample vessel and measuring the emitted radiation from the side of the excitation.

24. The method as in claim 13, wherein the presence of said extended oligonucleotide primer is determined in step (d) by excitating the reaction mixture sample in a sample vessel through the side of the uncovered liquid surface or through the bottom side of said sample vessel and measuring the emitted radiation from the side opposite to the side of the excitation.

25. The method as in claim 13, wherein the oligonucleotide primer to be extended comprises a fluorescent dye.

26. The method as in claim 25, wherein the determination step is carried out by measuring fluorescent intensity caused by an energy transfer relationship between said single-stranded DNA specific fluorescent dye and said fluorescent dye providing for fluorescence resonance energy transfer (FRET) between said single-stranded DNA specific fluorescent dye and said fluorescent dye.

## Patentansprüche

1. Verfahren zum Bestimmen der Anwesenheit von Verlängerungsprodukten, d.h. eines verlängerten Oligonucleotid-Primers, in einem Reaktionsgemisch, wobei ein Desoxynucleosid-5'-triphosphat (dNTP), das matrizen-abhängig in das 3'-Ende eines Oligonucleotid-Primers eingebaut werden soll, eine Markierung umfasst, umfassend die Schritte:
a) Verlängern des Oligonucleotid-Primers mit dem dNTP unter Bedingungen, die für die Primerverlängerung geeignet sind;
b) Kontaktieren des in Schritt a) erlangten verlängerten Oligonucleotid-Primers mit einem für Einzelstrang-DNA spezifischen Fluoreszenzfarbstoff, um an den verlängerten Oligonucleotid-Primer zu binden;
c) Bestimmen der Anwesenheit des verlängerten Oligonucleotid-Primers durch Messen der Intensität der Fluoreszenzemission nach Anregung.

2. Verfahren zum Bestimmen der Anwesenheit von Verlängerungsprodukten, d.h. eines verlängerten Oligonucleotid-Primers, in einem Reaktionsgemisch, wobei ein matrizenabhängig eingebautes Nucleotid in dem 3'-Ende des verlängerten Oligonucleotid-Primers eine fluoreszierende Nachweiseinheit umfasst, umfassend die Schritte:
a) Behandeln des Reaktionsgemischs, um einzelsträngige Verlängerungsprodukte zu erhalten, oder, wenn die Verlängerungsprodukte bereits einzelsträngig sind, direktes Anwenden von Schritt b);
b) Kontaktieren der einzelsträngigen Verlängerungsprodukte in dem Reaktionsgemisch mit einem für einzelsträngige DNA spezifischen Fluoreszenzfarbstoff, um nahe dem 3'-Ende des verlängerten Oligonucleotid-Primers zu binden, um eine Energietransferbeziehung zwischen der fluoreszierenden Nachweiseinheit und des für einzelsträngige DNA spezifischen Fluoreszenzfarbstoffs herzustellen, um Fluoreszenzresonanzenergietransfer (FRET) zwischen der fluoreszierenden Nachweiseinheit und dem für einzelsträngige DNA spezifischen Fluoreszenzfarbstoff zu ermöglichen;
c) Bestimmen der Anwesenheit des verlängerten Oligonucleotid-Primers durch Messen der Fluoreszenzintensität nach Anregung.

3. Verfahren nach Anspruch 2, wobei die Energietransferbeziehung auf Energietransfer zwischen einem Donor-Fluorophor und einem Akzeptor-Fluorophor basiert.

4. Verfahren nach Anspruch 2, wobei die Anwesenheit des verlängerten Oligonucleotid-Primers in Schritt c) bestimmt wird durch Messen mit mehr als einer Wellenlänge aus den Emissionsspektren der fluoreszierenden Nachweiseinheit und des für einzelsträngige DNA spezifischen Fluoreszenzfarbstoffs.

5. Verfahren nach Anspruch 2, wobei der für einzelsträngige DNA spezifische Fluoreszenzfarbstoff, der als Donor-Fluorophor agiert, ein Absorptionsmaximum bei etwa 498 nm und ein Emissionsmaximum bei etwa 518 nm hat, und wobei die fluoreszierende Nachweiseinheit, die als Akzeptor-Fluorophor agiert, ein Absorptionsmaximum bei etwa 596 nm und ein Emissionsmaximum bei etwa 620 nm hat.

6. Verfahren nach Anspruch 2, wobei nur ein Nucleotid in das 3'-Ende des verlängerten Oligonucleotid-Primers eingebaut wird.

7. Verfahren nach Anspruch 2, wobei das Reaktionsgemisch die für zyklische Minisequenzierung notwendigen Komponenten enthält.

8. Verfahren nach Anspruch 2, wobei das Reaktionsgemisch die für zyklische Minisequenzierung notwendigen Komponenten enthält und wobei die Matrize in dem Reaktionsgemisch ein PCR- oder RT-PCR-Produkt ist, das von einer Patientenprobe stammt.

9. Verfahren nach Anspruch 2, das verwendet wird, um Punktmutationen, z. B. SNPs, Allelismus oder den Genotyp menschlicher oder tierischer Wesen zu bestätigen.

10. Verfahren nach Anspruch 2, wobei das matrizenabhängig eingebaute Nucleotid im 3'-Ende des verlängerten Oligonucleotid-Primers von dATP, dCTP, dGTP oder dTTP abgeleitet ist, die in dem Reaktionsgemisch vorhanden sind und die mit einem Fluoreszenzfarbstoff markiert sind.

11. Verfahren nach Anspruch 2, wobei die Anwesenheit des verlängerten Oligonucleotid-Primers in Schritt c) bestimmt wird durch Anregen der Reaktionsgemischprobe in einem Probengefäß über die Seite der unbedeckten Oberfläche der Flüssigkeit oder über die Unterseite des Probengefäßes und Messen der emittierten Strahlung von der Seite der Anregung aus.

12. Verfahren nach Anspruch 2, wobei die Anwesenheit des verlängerten Oligonucleotid-Primers in Schritt c) bestimmt wird durch Anregen der Reaktionsgemischprobe in einem Probengefäß über die Seite der unbedeckten Oberfläche der Flüssigkeit oder über die Unterseite des Probengefäßes und Messen der emittierten Strahlung von der der Anregungsseite gegenüberliegenden Seite aus.

13. Verfahren zum Bestimmen der Anwesenheit von Verlängerungsprodukten, d.h. eines verlängerten Oligonucleotid-Primers, in einem Reaktionsgemisch, wobei ein matrizenabhängig eingebautes Nucleotid in dem 3'-Ende eines verlängerten Oligonucleotid-Primers eine Trennungseinheit umfasst, umfassend die Schritte:
a) Behandeln des Reaktionsgemischs, um einzelsträngige Verlängerungsprodukte zu erhalten oder, wenn die Verlängerungsprodukte bereits einzelsträngig sind, direktes Anwenden von Schritt b);
b) Abtrennen des verlängerten Oligonucleotid-Primers aus dem Reaktionsgemisch;
c) Kontaktieren des in Schritt b) abgetrennten verlängerten Oligonucleotid-Primers mit einem für Einzelstrang-DNA spezifischen Farbstoff zur Bindung an den verlängerten Oligonucleotid-Primer;
d) Bestimmen der Anwesenheit des verlängerten Oligonucleotid-Primers durch Messen der Intensität der Fluoreszenzemission nach Anregung.

14. Verfahren nach Anspruch 13, wobei der verlängerte Oligonucleotid-Primer in Schritt b) an einen festen Träger immobilisiert ist, der mit einem für die Trennungseinheit spezifischen Rezeptor beschichtet ist.

15. Verfahren nach Anspruch 13, wobei Biotin als Trennungseinheit benutzt wird und wobei der verlängerte Oligonucleotid-Primer in Schritt b) an einen festen Träger immobilisiert ist, der mit Streptavidin oder Avidin beschichtet ist.

16. Verfahren nach Anspruch 13, wobei die Anwesenheit des verlängerten Oligonucleotid-Primers in Schritt c) bestimmt wird durch Messen mit mehr als einer Wellenlänge aus dem Emissionsspektrum des Einzelstrang-DNA spezifischen Farbstoffs.

17. Verfahren nach Anspruch 13, wobei der für Einzelstrang-DNA spezifische Fluoreszenzfarbstoff ein Absorptionsmaximum bei etwa 498 nm und ein Emissionsmaximum bei etwa 518 nm hat.

18. Verfahren nach Anspruch 13, wobei nur ein Nucleotid in das 3'-Ende des verlängerten Oligonucleotid-Primers eingebaut ist.

19. Verfahren nach Anspruch 13, wobei das Reaktionsgemisch die für zyklische Minisequenzierung notwendigen Komponenten enthält.

20. Verfahren nach Anspruch 13, wobei das Reaktionsgemisch die für zyklische Minisequenzierung notwendigen Komponenten enthält und wobei die Matrize in dem Reaktionsgemisch ein PCR- oder RT-PCR-Produkt ist, das von einer Patientenprobe stammt.

21. Verfahren nach Anspruch 13, das verwendet wird, um Punktmutationen, z. B. SNPs, Allelismus oder den Genotyp menschlicher oder tierischer Wesen zu bestätigen.

22. Verfahren nach Anspruch 13, wobei das matrizenabhängig eingebaute Nucleotid in dem 3'-Ende des verlängerten Oligonucleotid-Primers von dATP, dCTP, dGTP oder dTTP abgeleitet ist, die in dem Reaktionsgemisch vorhanden sind und die mit Biotin markiert sind.

23. Verfahren nach Anspruch 13, wobei die Anwesenheit des verlängerten Oligonucleotid-Primers in Schritt d) bestimmt wird durch Anregen der Reaktionsgemischprobe in einem Probengefäß über die Seite der unbedeckten Oberfläche der Flüssigkeit oder über die Unterseite des Probengefäßes und Messen der emittierten Strahlung von der Seite der Anregung aus.

24. Verfahren nach Anspruch 13, wobei die Anwesenheit des verlängerten Oligonucleotid-Primers in Schritt d) bestimmt wird durch Anregen der Reaktionsgemischprobe in einem Probengefäß über die Seite der unbedeckten Oberfläche der Flüssigkeit oder über die Unterseite des Probengefäßes und Messen der emittierten Strahlung von der der Anregungsseite gegenüberliegenden Seite aus.

25. Verfahren nach Anspruch 13, wobei der zu verlängernde Oligonucleotid-Primer einen Fluoreszenzfarbstoff umfasst.

26. Verfahren nach Anspruch 25, wobei der Bestimmungsschritt ausgeführt wird durch Messen der Fluoreszenzintensität, die durch eine Energietransferbeziehung zwischen dem für einzelsträngige DNA spezifischen Fluoreszenzfarbstoff und dem Fluoreszenzfarbstoff verursacht wird, die Fluoreszenzenergietransfer (FRET) zwischen dem für Einzelstrang-DNA spezifischen Fluoreszenzfarbstoff und dem Fluoreszenzfarbstoff bereitstellen.

## Revendications

1. Procédé de détermination de la présence de produits d'extension, à savoir une amorce oligonucléotidique allongée, dans un mélange de réaction, dans lequel un désoxyribonucléoside (5'-)triphosphate (dNTP) à incorporer d'une manière dépendante d'une matrice dans la terminaison 3' d'une amorce oligonucléotidique comprend un marqueur, comprenant les étapes consistant à :
a) allonger ladite amorce oligonucléotidique avec ledit dNTP dans des conditions appropriées pour une extension d'amorce ;
b) amener ladite amorce oligonucléotidique allongée provenant de l'étape (a) au contact d'un colorant fluorescent spécifique d'un ADN monobrin pour une liaison à ladite amorce oligonucléotidique allongée;
c) déterminer la présence de ladite amorce oligonucléotidique allongée en mesurant l'intensité d'émission de fluorescence après excitation.

2. Procédé de détermination de la présence de produits d'extension, à savoir une amorce oligonucléotidique allongée, dans un mélange de réaction, dans lequel un nucléotide incorporé d'une manière dépendante d'une matrice dans la terminaison 3' de ladite amorce oligonucléotidique allongée comprend un fragment de détection fluorescent, comprenant les étapes consistant à :
a) traiter ledit mélange de réaction pour obtenir des produits d'extension monobrin, ou si les produits d'extension sont déjà de type monobrin, employer directement l'étape b) ;
b) amener les produits d'extension monobrin contenus dans ledit mélange de réaction au contact d'un colorant fluorescent spécifique d'un ADN monobrin pour une liaison à proximité de la terminaison 3' de l'amorce oligonucléotidique allongée de façon à former une relation de transfert d'énergie entre ledit fragment de détection fluorescent et ledit colorant fluorescent spécifique de l'ADN monobrin pour fournir un transfert d'énergie de résonance à fluorescence (FRET) entre ledit fragment de détection fluorescent et ledit colorant fluorescent spécifique de l'ADN monobrin ;
c) déterminer la présence de ladite amorce oligonucléotidique allongée en mesurant l'intensité de la fluorescence après excitation.

3. Procédé selon la revendication 2, dans lequel ladite relation de transfert d'énergie est fondée sur un transfert d'énergie entre un fluorophore donneur et un fluorophore accepteur.

4. Procédé selon la revendication 2, dans lequel on détermine la présence de ladite amorce oligonucléotidique allongée dans l'étape (c) au moyen d'une mesure avec plus d'une longueur d'onde à partir des spectres d'émission dudit fragment de détection fluorescent et dudit colorant fluorescent spécifique de l'ADN monobrin.

5. Procédé selon la revendication 2, dans lequel ledit colorant fluorescent spécifique de l'ADN monobrin, servant de fluorophore donneur, a un maximum d'absorption à environ 498 nm et un maximum d'émission à environ 518 nm, et ledit fragment de détection fluorescent, servant de fluorophore accepteur, a un maximum d'absorption à environ 596 nm et un maximum d'émission à environ 620.

6. Procédé selon la revendication 2, dans lequel seulement un nucléotide est incorporé dans la terminaison 3' de ladite amorce oligonucléotidique allongée.

7. Procédé selon la revendication 2, dans lequel ledit mélange de réaction contient les composants nécessaires pour un miniséquençage cyclique.

8. Procédé selon la revendication 2, dans lequel ledit mélange de réaction contient les composants nécessaires pour un miniséquençage cyclique et la matrice dans ledit mélange de réaction est un produit de PCR ou de RT-PCR provenant d'un échantillon d'un patient.

9. Procédé selon la revendication 2, qu'on utilise pour confirmer des mutations ponctuelles, par exemple des SNP, un allélisme ou le génotype d'un être humain ou animal.

10. Procédé selon la revendication 2, dans lequel ledit nucléotide incorporé d'une manière dépendante de la matrice dans la terminaison 3' de l'amorce oligonucléotidique allongée est dérivé de dATP, dCTP, dGTP ou dTTP présent dans ledit mélange de réaction et marqué avec un colorant fluorescent.

11. Procédé selon la revendication 2, dans lequel on détermine la présence de ladite amorce oligonucléotidique allongée dans l'étape (c) par excitation de l'échantillon du mélange de réaction dans une cuve d'échantillonnage du côté de la surface liquide non recouverte ou du côté inférieur de ladite cuve d'échantillonnage et en mesurant le rayonnement émis du côté de l'excitation.

12. Procédé selon la revendication 2, dans lequel on détermine la présence de ladite amorce oligonucléotidique allongée dans l'étape (c) par excitation de l'échantillon du mélange de réaction dans une cuve d'échantillonnage du côté de la surface liquide non recouverte ou du côté inférieur de ladite cuve d'échantillonnage et en mesurant le rayonnement émis du côté opposé au côté de l'excitation.

13. Procédé de détermination de la présence de produits d'extension, à savoir une amorce oligonucléotidique allongée, dans un mélange de réaction, dans lequel un nucléotide incorporé d'une manière dépendante de la matrice dans la terminaison 3' d'une amorce oligonucléotidique allongée comprend un fragment de séparation, comprenant les étapes consistant à :
a) traiter ledit mélange de réaction de façon à obtenir des produits d'extension monobrin, ou si les produits d'extension sont déjà de type monobrin, employer directement l'étape (b) ;
b) séparer ladite amorce oligonucléotidique allongée dudit mélange de réaction;
c) amener ladite amorce oligonucléotidique allongée séparée dans l'étape (b) au contact d'un colorant spécifique de l'ADN monobrin pour une liaison à ladite amorce oligonucléotidique allongée;
d) déterminer la présence de ladite amorce oligonucléotidique allongée en mesurant l'intensité de la fluorescence après excitation.

14. Procédé selon la revendication 13, dans lequel ladite amorce oligonucléotidique allongée est immobilisée dans l'étape (b) sur un support solide revêtu d'un récepteur spécifique pour ledit fragment de séparation.

15. Procédé selon la revendication 13, dans lequel on utilise de la biotine comme ledit fragment de séparation, et ladite amorce oligonucléotidique allongée est immobilisée dans l'étape (b) sur un support solide revêtu de streptavidine ou d'avidine.

16. Procédé selon la revendication 13, dans lequel on détermine la présence de ladite amorce oligonucléotidique allongée dans l'étape (c) au moyen d'une mesure avec plus d'une longueur d'onde à partir du spectre d'émission dudit colorant spécifique de l'ADN monobrin.

17. Procédé selon la revendication 13, dans lequel ledit colorant fluorescent spécifique de l'ADN monobrin a un maximum d'absorption à environ 498 nm et un maximum d'émission à environ 518 nm.

18. Procédé selon la revendication 13, dans lequel seulement un nucléotide est incorporé dans la terminaison 3' de ladite amorce oligonucléotidique allongée.

19. Procédé selon la revendication 13, dans lequel ledit mélange de réaction contient les composants nécessaires pour un miniséquençage cyclique.

20. Procédé selon la revendication 13, dans lequel ledit mélange de réaction contient les composants nécessaires pour un miniséquençage cyclique, et la matrice dans ledit mélange de réaction est un produit de PCR ou de RT-PCR provenant d'un échantillon d'un patient.

21. Procédé selon la revendication 13, que l'on utilise pour confirmer des mutations ponctuelles, par exemple des SNP, un allélisme ou le génotype d'un être humain ou animal.

22. Procédé selon la revendication 13, dans lequel ledit nucléotide incorporé d'une manière dépendante de la matrice dans la terminaison 3' de l'amorce oligonucléotidique allongée est dérivé de dATP, dCTP, dGTP ou dTTP présent dans ledit mélange de réaction et marqué avec de la biotine.

23. Procédé selon la revendication 13, dans lequel on détermine la présence de ladite amorce oligonucléotidique allongée dans l'étape (d) par excitation de l'échantillon du mélange de réaction dans une cuve d'échantillonnage du côté de la surface liquide non recouverte ou du côté inférieur de ladite cuve d'échantillonnage et en mesurant le rayonnement émis du côté de l'excitation.

24. Procédé selon la revendication 13, dans lequel on détermine la présence de ladite amorce oligonucléotidique allongée dans l'étape (d) par excitation de l'échantillon du mélange de réaction dans une cuve d'échantillonnage du côté de la surface liquide non recouverte ou du côté inférieur de ladite cuve d'échantillonnage et en mesurant le rayonnement émis du côté opposé au côté de l'excitation.

25. Procédé selon la revendication 13, dans lequel l'amorce oligonucléotidique à allonger comprend un colorant fluorescent.

26. Procédé selon la revendication 25, dans lequel on effectue l'étape de détermination en mesurant l'intensité de la fluorescence causée par une relation de transfert d'énergie entre ledit colorant fluorescent spécifique de l'ADN monobrin et ledit colorant fluorescent fournissant un transfert d'énergie de résonance à fluorescence (FRET) entre ledit colorant fluorescent spécifique de l'ADN monobrin et ledit colorant fluorescent.
